# EUROPEAN PATENT APPLICATION

(11) **EP 4 169 526 A1**
(43) Date of publication of application: **26.04.2023**
(21) Application number: 21203999.4
(22) Date of filing: 21.10.2021
(51) Int. Cl.: A61K 38/50, A61K 35/407, A61K 38/28, A61K 38/53, A61K 38/54, A61P 35/00

(54) **ARGINASE BASED CANCER THERAPY**

(71) Applicant: Kyon Biotech AG, 8005 Zürich (CH)
(72) Inventor: CVETKOVIC, Goran, 8645 Rapperswil-Jona (CH); TEPIC, Slobodan, 7252 Klosters Dorf (CH)
(74) Representative: Weiss, Wolfgang

(57) **Abstract**

The present invention relates to a medicament comprising an arginine decomposing enzyme, and a citrulline converting enzyme useful for the treatment of cancer.

## Description

The present invention relates to a medicament comprising an arginine decomposing enzyme, and a citrulline converting enzyme useful for the treatment of cancer.

### Background

Depletion of L-arginine (in the following arginine) has been shown of utility in treating some cancers such as hepatocellular carcinoma and melanoma, and based on *in vitro* work, probably many others. The use of arginine depleting enzymes such as arginase in cancer therapy has e.g., been described by Shen et al. (Cell Death & Disease 8 (2017), e2720), Zou et al. (Biomedicine & Pharmacotherapy 118 (2019), 109210), Al-Koussa et al. (Cancer Cell International 20 (2020) Article number 150) and Zhang et al. (Cancer Letters 502 (2012), 58-70), the contents of which are herein incorporated by reference.

Use of arginine depleting enzymes is necessary, but our own research has shown it not being sufficient to cause and maintain systemic, deep depletion of arginine needed to cause rapid, selective killing of cancer cells.

The use of an insulin/glucose clamp in parallel with enzymatic degradation of arginine makes the task of deep arginine depletion much more manageable. Insulin is a growth factor and thus promotes protein synthesis and inhibits protein breakdown. This is of crucial importance when the task is removing an amino acid from circulation, and particularly of removing arginine, which is a semi-essential amino acid under tight homeostatic control.

An increase of vascular permeability by insulin also helps in getting therapeutic enzymes into interstitial fluid space, closer to where most cancerous cells reside. Finally, insulin may also play a role in transporting arginine degrading enzymes into cancerous cells by stimulating endocytosis.

However, as the inventors' research has shown, even with the use of an insulin/glucose clamp, free arginine levels on the order of 5 to 10 µM were obtained compared to the normal plasma concentration of about 100 µM. At these free arginine levels, cells, cancerous and healthy, will not proliferate but still can survive for prolonged periods. The target for arginine concentration to result in rapid killing of cancer cells is 1 µM or less.

The inventors have also attempted inhibition of citrulline production in the intestines but none of the many approaches tested in experimental dogs have resulted in the satisfactory reduction of plasma citrulline.

It was an object of the invention, to provide means for improving the depletion of free arginine in the blood of subject, particularly of a human cancer patient. More particularly, it was an object of the invention to overcome disadvantages associated with previous treatment schedules involving amino acid depletion with, e.g., administration of PEGylated arginase or PEGylated arginine deiminase (ADI), which are currently investigated in over 30 clinical trials for cancer treatment.

### Description of the invention

The inventors have found that systemic delivery of arginase (ARG) and argininosuccinate synthase (ASS) in partially purified liver extracts prepared by low-temperature protocols has led to a reduction of free plasma arginine to below detection levels. This result is the basis of the present invention relating to the use of ASS together with ARG in order to obtain a deep depletion of free arginine levels in circulating blood.

The present invention relates to the depletion of arginine by enzymatic means for use in medicine, particularly for the treatment of cancer. Enzymatic decomposition of arginine by e.g., an arginase (ARG) was found to be necessary but not sufficient for a deep, systemic depletion of arginine. A concurrent removal of citrulline on its passage from intestines, the main organ for citrulline synthesis, to kidneys where citrulline is converted to arginine, by a citrulline-converting enzyme, e.g., argininosuccinate synthase (ASS) enables systemic reduction of free arginine to a concentration below detection by standard amino acids analysis. This leads to a rapid killing of cancerous cells of many if not all cancer types. Healthy dividing cells respond by exiting into G₀ phase and very few suffer terminal damage.

A first aspect of the invention relates to a medicament comprising a first active agent, which is an arginine-decomposing enzyme, and a second active agent, which is a citrulline-converting enzyme.

The medicament of the invention is suitable for use in human medicine and veterinary medicine, e.g. for the treatment of dogs. Administration of the medicament in a therapeutically active dose will lead to a deep depletion of arginine levels in blood.

In certain embodiments, administration of the medicament lowers the arginine level to about 20 µM or less for a time of at least 12 h or even for at least 24 h. Preferably the arginine level is lowered to about 10 µM or less, more preferably the arginine level is lowered to about 5 µM or less for a time of at least 12 h or even for at least 24 h, and most preferably the arginine level is lowered about 1 µM or less for a time of at least 12 h or even for at least 24 h as measured by in the venous blood plasma.

In certain embodiments, administration of the medicament lowers the citrulline level in the venous blood plasma about two to five-fold compared to the citrulline level without administration of the medicament. The citrulline concentration in human plasma of healthy individuals is about 30 to 50 µM. Observations from human patients with advanced hepatocellular carcinoma that the inventors participated in treating with arterial occlusion of the liver suggest that lowering citrulline level two to five-fold is sufficient to lower arginine level to the target of less than 1 µM. In dogs, the normal citrulline concentration is about two times higher than in humans but the same reduction of two to five times was correlated with successful arginine depletion.

The arginine-decomposing enzyme and/or the citrulline-converting enzyme may be a purified or partially purified polypeptide from a natural source, e.g. body tissue such as liver, or a body fluid. Alternatively, the arginine-decomposing enzyme and/or the citrulline-converting enzyme may be a recombinant polypeptide prepared from a genetically engineered host cell, e.g. a prokaryotic cell such as *E. coli* or a eukaryotic cell such as a yeast, insect or mammalian cell. The host cell may be transfected with a nucleic acid molecule encoding an enzyme of the present invention. The enzyme may be produced by cultivating the host cell and obtaining the protein from the host cell or from the culture media.

In certain embodiments, the first active agent and the second active agent are present as an at least partially purified mammalian liver extract. A preferred method for obtaining a suitable liver extract is described in the Examples. In certain embodiments, the first active agent and the second active agent are recombinant polypeptides.

In certain embodiments, the arginine-decomposing enzyme is an arginase (ARG), i.e. an enzyme, which catalyzes the hydrolysis of L-arginine to L-ornithine and urea (EC. 3.5.3.1). Typically, the arginase is a mammalian arginase, e.g. a human arginase including any enzymatically active fragment and derivative thereof. In particular embodiments, the arginase is selected from human arginase-1 (liver arginase or ARG1) (UniProt-P05089) or human arginase 2 (kidney arginase or ARG2) (UniProt-P78540) including any enzymatically active fragment and derivative thereof. In even more particular embodiments, the arginase is human ARG1.

Human ARG1 is a 322 amino acid long polypeptide with a predicted molecular weight of 34,735 Da. It may be present in a monomeric or multimeric, e.g., trimeric form. Preferably, it is present in a monomeric form, which is the native form in the liver (personal communication from Prof. M. Ikemoto, U. of Kyoto, who was the first to clone ARG1). The preparation of recombinant ARG1 in *E. coli* is described by Ikemoto et al. in: Expression of human liver arginase in Escherichia coli. Purification and properties of the product. Biochem J. 1990 Sep 15;270(3):697-703. doi: 10.1042/bj2700697. PMID: 2241902; PMCID: PMC1131788; the content of which is herein incorporated by reference.

In certain embodiments, the arginase is a recombinant human ARG1 including any enzymatically active fragment and derivative thereof. In particular embodiments, the arginase is an unPEGylated polypeptide, i.e. it not conjugated to a polyethylene glycol moiety.

A modification of human ARG1 to replace manganese with cobalt and to shift the optimum pH to that of plasma is also particularly suitable according to the present invention (Stone EM, Glazer ES, Chantranupong L, et al. Replacing Mn(2+) with Co(2+) in human arginase enhances cytotoxicity toward I-arginine auxotrophic cancer cell lines [published correction appears in ACS Chem Biol. 2010 Aug 20;5(8):797]. ACS Chem Biol. 2010;5(3):333-342. doi:10.1021/cb900267j), the content of which is herein incorporated by reference.

In certain embodiments, arginase, e.g., human ARG1 is administered as an arginase-insulin fusion protein as described in co-owned application EP 211976781.4, the content of which is herein incorporated by reference.

In certain embodiments, the citrulline-converting enzyme is an argininosuccinate synthase (ASS), i.e. an enzyme, which catalyzes the conversion of L-aspartate and L-citrulline to L-argininosuccinate (EC 6.3.4.5). Typically, the ASS is a mammalian ASS, e.g. a human ASS including any enzymatically active fragment and derivative thereof. In particular embodiments, the ASS is human argininosuccinate synthase (ASS1) (UniProt-P00966) including any enzymatically active fragment and derivative thereof.

Human ASS1 is a 412 amino acids long polypeptide with a predicted molecular weight of 46,530 Da. It may be present in a monomeric or multimeric, e.g., tetrameric form. Preferably, it is present in a tetrameric form, which is its native active form in the human liver.

In certain embodiments, the ASS is a recombinant human ASS1 including any enzymatically active fragment and derivative thereof. In certain embodiments, the ASS is a PEGylated polypeptide. In other embodiments, the ASS is an unPEGylated polypeptide.

The medicament of the present invention comprises the first active agent and the second active agent optionally together with pharmaceutically acceptable excipients, e.g., selected from buffers, salts, and/or stabilizers.

The medicament may be a single pharmaceutical preparation comprising both the first and the second active agent. Alternatively, the medicament may be a combination of two separate pharmaceutical preparations, one comprising the first active agent and the other one comprising the second active agent.

The pharmaceutical preparation may be a liquid pharmaceutical preparation comprising the active agent(s) in dissolved or suspended form ready for use. Alternatively, the pharmaceutical preparation may be a solid pharmaceutical preparation comprising the active agent(s) in lyophilized or freeze-dried form for reconstitution with a suitable solvent, e.g., an aqueous solvent.

The medicament is administered in therapeutically effective doses of the first and the second active agent. In certain embodiments, the first and the second active agent are administered in similar doses based on enzymatic activity, e.g., wherein the ratio of enzymatic activities of ARG to ASS is within the range of about 0.2:1 to about 1:5, particularly of about 0.5:1 to about 1:2 and more particularly about 1:1 taking in account that Vmax for ARG is 1000 times higher than Vmax for ASS, but Km of ASS is 1000 times lower, so at micromolar concentrations of substrates, the conversion rates are about the same per mg of proteins.The basis for the same dose (expressed as enzymatic activity at concentrations of respective substrates normally present in plasma) measured at physiological concentrations of respective substrates) for the enzymes, e.g., ARG1 and ASS1, arises from their function in the urea cycle. The inventors have successfully prepared and tested in dogs partially purified extracts of pig liver. The goal of lowering plasma arginine to below detection was achieved with the activity of the tandem as present in the liver where both enzymes are active mostly within the urea cycle and thus, in the steady state, convert the same number of molecules of arginine and citrulline in unit time.

In certain embodiments, the therapeutically effective daily dose of an arginase, e.g., ARG1, in a dog is about 300 and about 6000 U/kg/day, preferably about 1000 and about 4500 U/kg/day, and most preferably 3000 U/kg/day as determined by experimental work *in vivo.* For humans, a therapeutically effective daily dose is about 150 to about 3000 U/kg/day, preferably about 500 to about 2000 U/kg/day, and most preferably about 1500 U/kg/day. With a specific activity of recombinant ARG1 of about 1000 U/mg of protein a preferred therapeutically effective daily dose in a dog is about 0.3 to about 6.0 mg/kg/day and in a human about 0.15 to about 3.0 mg/kg/day.

In certain embodiments, the therapeutically effective daily dose of an ASS, e.g., ASS1, in a dog is about 0.3 and about 6 U/kg/day, preferably about 1 and about 4.5 U/kg/day, and most preferably 3 U/kg/day. For humans, a therapeutically effective daily dose is about 0.15 to about 3 U/kg/day, preferably about 0.5 to about 2 U/kg/day, and most preferably about 1.5 U/kg/day. With a specific activity of recombinant ASS1 of about 1 U/mg of protein a preferred therapeutically effective daily dose in a dog is about 0.3 to about 6.0 mg/kg/day and in a human about 0.15 to about 3.0 mg/kg/day.

In certain embodiments, the dose is adapted based on the measurement of arginine and/or citrulline levels in the blood of the subject to be treated. In particular embodiments, the arginine and/or citrulline levels are measured in plasma of the venous blood.

The medicament is typically administered parenterally, e.g., by injection, or preferably by infusion over a suitable period, for example, over a period from several, e.g. at least about 2 hours, or about 6 hours, or even continuously during 1 day up to 6 days, depending on the type of cancer being treated.

Administration of the medicament may be accompanied by certain measures to compensate side-effects of arginine depletion such as infusion of a nitric oxide (NO) donor, e.g., sodium nitroprusside (SNP), and/or a pressor peptide, e.g., a vasopressin, to balance NO-induced vasodilation. Arginine is the only precursor for synthesis of short-lived NO. All pressor peptides contain arginine and are short-lived. Co-infusion of Iloprost, a prostacyclin analog has also been found useful in the maintenance of thrombocytes.

The medicament is useful for the treatment of cancer including a blood cancer or a solid cancer particularly selected from hepatocellular carcinoma, melanoma, colon carcinoma, leukemia, lymphoma, osteosarcoma, soft tissue sarcoma, mast cell tumor, pancreatic cancer, lung cancer, and breast cancer. The medicament is also useful for the treatment of cancer metastases, which are disseminated within the patient's body.

The inventors have found that the efficacy of the medicament may be increased by assisting extravasation of arginase, i.e. the transport from the vascular system into the interstitial fluid. Rapid extravasation of ARG1 in its monomeric form, facilitated by insulin, prevents its elimination by glomerular filtration as described in co-owned application PCT/EP2021/072616 the content of which is herein incorporated by reference.

The target compartment for the arginase is the interstitial volume. This is where most cancer cells reside - only a small fraction of cancer cells are found in the blood at any stage of the disease, even in blood cancers. Enzymatic activity confined to the vascular system cannot efficiently reduce concentrations of amino acids in extravascular volume to very low levels because of the constant influx of amino acids from protein breakdown under homoeostatic mechanisms. The mass transport between intravascular and interstitial fluid (in both directions) is limited by diffusion and modest convection, the rates of which are simply too low when, e.g., arginine is to be systemically reduced into µM range.

It is worth noting that all current clinical trials with arginine depletion as a modality for cancer treatment are conducted with PEGylated enzymes (arginase or arginine deiminase) with very large molecular weights that make extravasation extremely limited and thus does not allow for deep systemic arginine depletion needed to kill disseminated cancer.

In certain embodiments of the invention, extravasation of arginase is enhanced by co-administration of the medicament together with an insulin, preferably as an insulin glucose clamp, i.e. by co-administration of an insulin and glucose. The insulin may be administered by infusion at a predetermined dose rate, e.g. at a predetermined constant dose rate. For humans, the insulin may be administered at a dose rate from about 1.5 to about 6 I.U./kg body weight/day, preferably from about 2 to about 4 I.U./kg body weight//day, more preferably about 3 I.U./kg body weight//day. For dogs, the insulin may be administered at a higher dose rate from about 3 to about 12 I.U./kg body weight//day, preferably from about 4 to about 8 I.U./kg body weight//day, more preferably about 6 I.U./kg body weight//day.

The insulin may be any type of natural or recombinant insulin or insulin analogue suitable for applying an insulin-glucose clamp. Preferably, the insulin is a rapid acting insulin or a short acting insulin, more preferably a rapid acting insulin. Examples of rapid acting insulin are insulin lispro, insulin aspart or insulin glulisine. Examples of short acting insulins are regular insulin or insulin velosulin.

According to the present invention, the insulin may be co-administered with glucose. Preferably, glucose is administered by infusion. In this context, it is noted that the term "glucose" as used herein also includes a glucose containing oligosaccharide or polysaccharide capable of releasing glucose into the blood, for example, any type of dextrose, such as partial hydrolysis products from starch or maltodextrin. The administration of glucose may be adjusted to maintain the plasma glucose level within normal, physiological concentrations, maintain a sufficient glucose level of e.g., from about 4.0 to about 10 mM. This may require delivery of about 10 g glucose/kg body weight/day for dogs at an insulin infusion dose rate of about 6 I.U./kg body weight/day; or delivery of about 5 g glucose /kg body weight/day for humans at an insulin infusion dose rate of about 3 I.U./kg body weight/day. This may be achieved by an infusion of a 10% glucose solution into a peripheral vein of an average person, started at a rate of about 150 ml/h for the middle dose rate of insulin (3 I.U./kg/day) and adjusted as needed. Dextrose, with an equivalent rate of infusion, can be used instead of glucose.

The delivery rate of a glucose solution can be controlled by a simple drip method from an infusion bag or bottle of e.g. 250, or 500 ml volume. Non-invasive monitoring of glucose can be performed by e.g. *FreeStyle Libre* from Abbott Laboratories.

Co-administration of insulin and glucose may start before, at or after administration of the medicament. Typically, co-administration of insulin and glucose is performed for at least about 3 h, at least about 6 h, for at least about 12 h or at least about 24 h and up to several days depending on the type and administration route of the medicament

Administration of glucose may be accompanied by administration of a potassium salt such as KCI to compensate for potassium ions entering cells if a high dose of glucose is administered. Further, the treatment may be supported by concomitant administration of essential amino acids, electrolytes, fluids and/or antibiotics.

In still further embodiments, extravasation of arginase is enhanced by using as the first active agent a fusion protein of arginase and insulin as described in co-owned application EP 211976781.4, preferably accompanied by co-administration of glucose.

By contrast, the role of ASS is very different. Arginine is a semi-essential amino acid and the only known synthetic pathway of relevance is the so-called intestinal-renal axis, whereby citrulline is produced in the intestines and then converted to arginine in the kidneys, the transfer between the two organs effected via blood. Thus, prolonged maintenance and circulation time for ASS may be useful.

There are many publications in the last years stating that only cancer cells that are auxotrophic for arginine are suitable targets for attack by arginine depletion. This argument misses the point that all cells, cancerous and healthy are auxotrophic for arginine. It is only on the systemic, whole-body level that the requirement for arginine is met by the intestinal-renal axis, i.e., by a synthetic pathway served by two distinct organs. All cells will sooner or later die if depleted for arginine. Some cancers can substitute citrulline for arginine, but the citrulline concentration required is very high, many times higher than physiological levels (Wheatley, D, and Campbell, E, Arginine deprivation, growth inhibition and tumour cell death: 3. Deficient utilisation of citrulline by malignant cells, V. 89, 10.1038/sj.bjc.6601134, British journal of cancer). Ornithine also cannot substitute for arginine (Wells JW, Evans CH, Scott MC, Rütgen BC, O'Brien TD, Modiano JF, Cvetkovic G, Tepic S, Arginase treatment prevents the recovery of canine lymphoma and osteosarcoma cells resistant to the toxic effects of prolonged arginine deprivation. PLoS One. 2013;8(1):e54464. doi: 10.1371/journal.pone.0054464. Epub 2013 Jan 24. PMID: 23365669; PMCID: PMC3554772.).

The point is that cancer cells will die much quicker than healthy cells, i.e., the selectivity of arginine depletion for cancer cells is expressed in terms of time to cellular death. This, of course, does not deny in any way the existence of genetic differences between healthy and transformed cells, but simply states that the ultimate expression of the sum of any of these differences translates into metabolic needs of cancer being higher than for healthy cells. Arginine is the best target because it is a substrate in many biosynthetic pathways other than protein synthesis. Exceptionally high requirement for arginine thus leads to its rapid self-depletion, especially in faster growing and dividing cells, the hallmark of cancers.

To effectively inhibit this main source of arginine on the systemic level, the present invention relies on interrupting citrulline flux from intestines to kidneys, by loading the vascular system with a citrulline-converting enzyme. While inhibiting the synthesis of citrulline in the intestines appears to be a better approach, the inventors have failed, as already stated, to accomplish that goal despite many approaches tested, including elevation of lactate and use of gly-gly-PALO inhibitor of ODC enzyme in the pathway of citrulline synthesis (Cvetkovic, G, Inhibition of endogenous sources of arginine in arginine depleted dogs, Inaugural-Dissertation, U. of Zurich, 2010).

To inhibit the intravascular flux of citrulline from the intestines to kidneys it is important to reduce extravasation of the ASS enzyme, hence it is preferred to prepare it and use it in its tetrameric form, which also is its native form in the liver. Recombinant ASS1 expressed in *E. coli* may thus be biochemically treated to facilitate its constitution into a tetramer. Unlike in the case of ARG1, PEGylation of ASS, particularly of ASS1 is feasible for it can increase its intravascular half-life.

Experimental *in vivo* work with healthy dogs and dogs with naturally occurring cancers that led to this invention was carried out mostly at the Animal Hospital of the School of Veterinary Medicine, U. of Zurich, and at several veterinary clinics in Switzerland and abroad, starting in 1996. Over a hundred sessions of continuous treatment lasting up to 6 days were carried out on as many dogs in pursuit of sustained, deep, systemic depletion of arginine. During a short period of collaboration, that started in late 2000 with a human oncologist in Hong Kong, four human patients with advanced hepatocellular carcinoma were treated with endogenous release of arginase caused by otherwise conventional, palliative treatment by occlusion of arterial liver perfusion. Very positive outcomes in those patients led to one of the two currently most clinically active projects with PEGylated arginine depleting enzymes.

The present inventors have been deeply involved in all aspects of these arginine depletion projects for over 25 years and by careful examination of failures and successes to achieve full systemic depletion have devised a protocol for preparation of a partially purified liver extract, which has retained activity of the tandem of enzymes needed to lower systemic arginine to below detection. To support the claims of this invention a brief description of the steps that have led to it are given below.

### Figure Legends

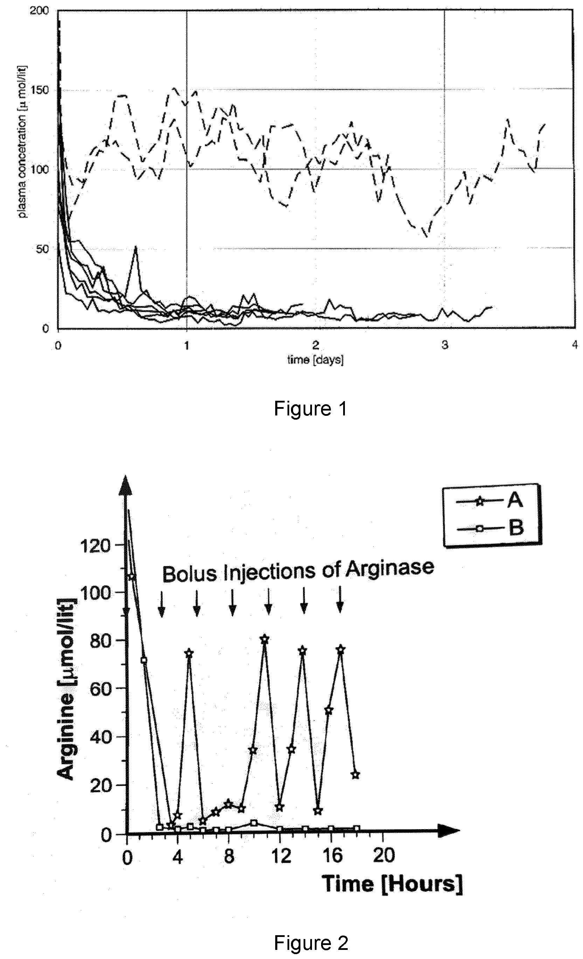

Figure 1: Effect of selective dialysis on the plasma arginine concentration in dogs. Dashed lines show plasma arginine concentration in 2 dogs without administration of insulin. Solid lines show plasma arginine concentration in 6 dogs with infusion of insulin. Combined selective dialysis and insulin (glucose provided via dialysis) show a reduction about ten-fold from about 100 µM to about 10 µM.
Figure 2: Administration of autologous crude liver extract rich in arginase by bolus infusions every 3 hours, during 18 hours in total without insulin/glucose clamp (curve A) and with insulin/glucose clamp (curve B). Without insulin/glucose clamp, plasma arginine level dropped to near zero and returned to a normal level before the next bolus infusion 3 h later. With an insulin/glucose clamp, plasma arginine was lowered to below detection and held there for 18 h.

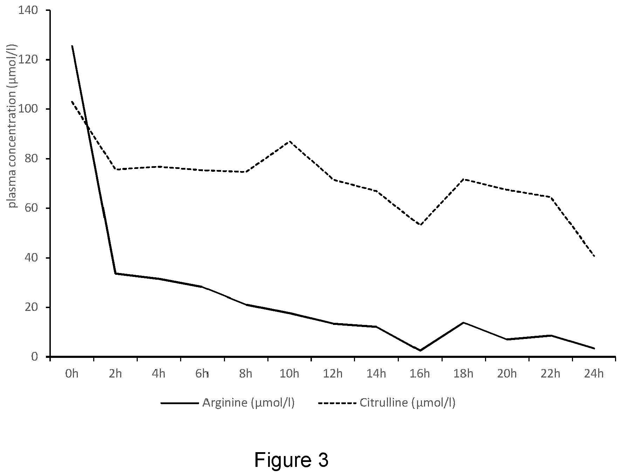

Figure 3: Administration of PEGylated recombinant human liver arginase combined with potential inhibitors of the intestinal-renal axis and of systemic protein breakdown. Pre-terminal experiments during one day of continuous infusions into anesthetized dogs. Plots show plasma concentrations of arginine and citrulline achieved by elevating lactate by co-infusion of lactic acid and sodium lactate.

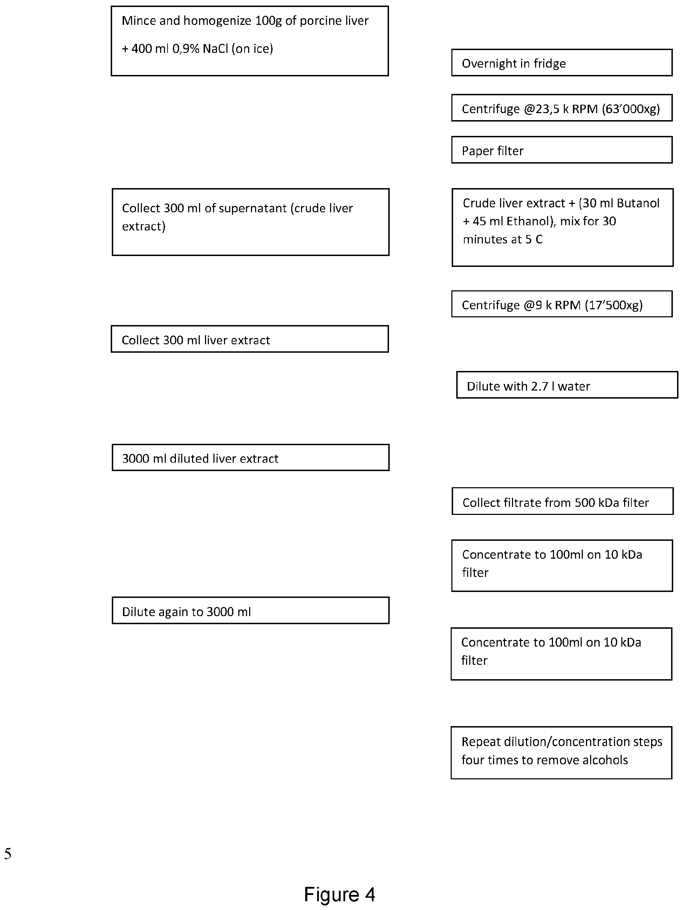

Figure 4: Chart with steps for low-temperature partial purification of a (porcine) liver extract with ultrafiltration and selective removal of hemoglobin, the main contaminant, by solvent-mediated precipitation.

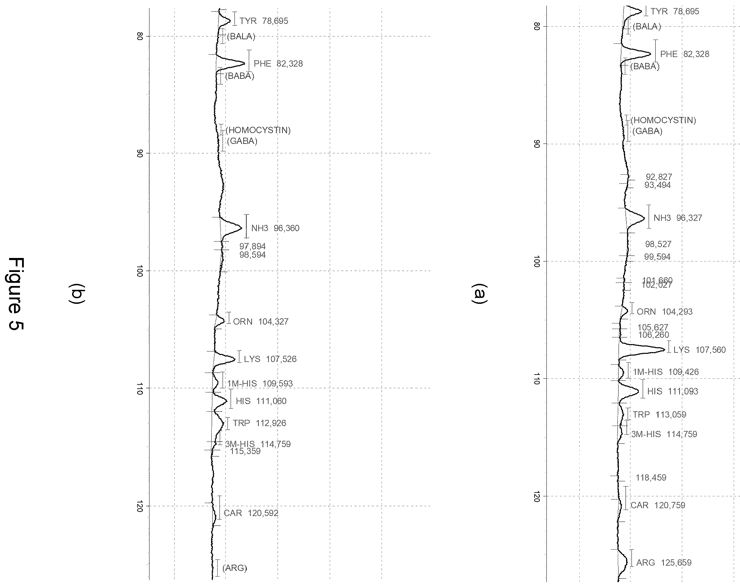

Figure 5: Chromatograms from an amino acids analyzer showing the section of the plot with a normal level of arginine in a dog, (a), and the same section of the plot in the same dog treated by low-temperature partially purified porcine liver extract, (b), on the third day of treatment. During the first, the second, and the fourth day of the treatment, with the high-temperature purification, plasma arginine concentration could not be lowered below 20 µM.

### Experimental observations and conclusions therefrom

### Extracorporeal removal of plasma arginine

Removal of low molecular constituents of blood plasma by hemodialysis is a routine procedure used on hundreds of thousands of patients several times per week for decades. This procedure was the original approach to deplete arginine levels in blood by the present inventors.

In the first experiments with healthy, conscious dogs, blood was circulated via central venous catheters through a closed extracorporeal circuit with partially purified bovine arginase contained in the outer envelope of a conventional, high flux (32 kDa cutoff) dialysis filter. Amino acids were measured on the inlet and outlet of the filter. Even after a couple of days of continuous blood circulation and near-total conversion of arginine to ornithine on the passage through the filter, there was no reduction of arginine in the blood plasma at the inlet to the filter.

This failure prompted a change in the approach to using selective amino acid removal by extracorporeal blood treatment. Conventional hemodialysis uses dialyzing fluid with only electrolytes, pH-controlling substances, and glucose. During a session on dialysis (about 6 hours with standard cutoff filters at 10 kDa; about 2 hours with high flux filters with cutoff at 32 kDa), all plasma molecules with a molecular weight below the cutoff were washed out. That includes all amino acids. Despite these losses, plasma concentrations of amino acids remained relatively high having triggered a homeostatic response by protein breakdown. The unfortunate consequence of this dynamic may cause an overshoot in amino acids post-dialysis and in turn their breakdown by the liver, presenting a new burden on the failing kidneys. A small percentage of patients on dialysis, so called "shrinking patients", fail to control protein breakdown and die from the sequelae.

To enable longer, measured in days, duration of continuous dialysis to remove arginine, the inventors prepared a dialyzing fluid by adding all known small molecular weight water-soluble components of blood plasma (52 in number) except for the targeted amino acid, e.g., arginine. Shown in Figure 1 with dashed lines are measured arginine concentrations in two dogs. Only a minor reduction was possible due to massive systemic protein breakdown.

However, by the concurrent delivery of insulin, the arginine concentration could be lowered to and maintained at about 10% of normal physiological level as shown by solid lines in Figure 1.

Despite this partial success, the extracorporeal blood treatment was abandoned once a sample of the lymphatic fluid showed arginine concentration at twice its normal level in blood plasma. The molecular exchange between the blood and extravascular fluid was simply no match for the influx of amino acids from protein breakdown, mostly of muscle proteins.

### Infusion of autologous crude liver extract

The failure of selective dialysis to lower plasma arginine to the target of less than 1 µM, prompted another approach - to enzymatic degradation of arginine. The use of asparaginase in treating Acute Lymphocytic Leukemia (ALL) in children was a uniquely successful predicate in oncology. In fact, the success, but also limited indications of asparaginase in treating blood cancers only, prompted several major efforts to discover enzymes that would target essential amino acids and be useful in treating solid tumors. Several of these projects are still ongoing, with arginine depletion by arginase or arginine deiminase in the forefront.

Another set of observations from clinical approaches to treating cancer provided a crucial link to the approach taken by present inventors. The limiting problem of prolonged (days in duration) extracorporeal blood treatment was blood clotting. As ultimately understood by the inventors, the clotting in all extracorporeal treatments of blood is caused by depletion of nitric oxide (NO), synthesized from arginine by mostly constitutive nitric oxide synthase enzyme (eNOS) in the endothelial cells. Prostacyclin is another crucial signal molecule needed to prevent the irreversible activation of thrombocytes. Both NO and prostacyclin are small short-lived molecules missing (not produced, even removed) in the extracorporeal tubing and the filter. Activated thrombocytes form blood clots in the filter and, with even worse consequences, in the capillary beds in the body, including in the kidneys. A typical thrombocyte count post a dialysis session is only half of that pre-dialysis.

The same cascade of events is triggered by treating colon cancer metastatic lesions in the liver by cryoablation. Blood clotting is common morbidity in many patients treated by this method. In rare cases, it can even lead to death due to disseminated intravasal clotting (DIC). As the inventors have demonstrated by performing liver cryoablation in experimental dogs, the path to clotting is driven by the release of arginase from ablated liver tissue, resulting in depletion of arginine and hence NO - locally and even systemically.

However, cryoablation of liver tissue (by freezing several ball-shaped lesions) caused only a temporary reduction of systemic arginine concentrations, which prompted another approach - the use of i.v. infusion of a crude autologous extract from a partially resected liver.

Results from two dogs treated with crude extract are shown in Figure 2 (animals were anesthetized during a 24-hour pre-terminal period). Bolus injections of the extract with arginase activity of 3000 U/kg/day caused a rapid reduction of plasma arginine, with an equally rapid return to a normal level before the next infusion 3 hours later (plot A). However, with an insulin/glucose clamp administered continuously during the experiment, plasma arginine was lowered to below detection (< 1 µM) and held there during most of the 18 hours of infusion time (plot B). Four different dosages were used in two dogs each. With 3000 U/kg/day and 1000 U/kg/day, arginine was held at zero. With 300 U/kg/day and 100 U/kg/day arginine was reduced but not to below detection.

### Conventional liver extract purification for the enrichment of arginase carried out at a high temperature

These early successes with crude liver extracts were followed by years of failures to reproduce these results relying on published methods of liver arginase purification. Compared to most liver proteins, mostly enzymes, arginase is heat-stable and that feature has been utilized in all purification schemes published in biochemical literature. A typical, very simple, and effective step in purification comprises 10 min of homogenate mixing at 65°C. A very large fraction of other proteins will precipitate and can be removed by simple centrifugation. Even with arginase activity maintained at expected levels, when used *in vivo,* these extracts could not reproduce the previous results obtained with crude liver extracts. For most of these experiments, the inventors used porcine liver, which potentially could have contributed to the failures due to a non-specific immune response. That, in due time, was ruled out.

The focus was then shifted to inhibiting the intestinal-renal axis with already mentioned *in vivo* work on inhibition of citrulline synthesis in the intestines. Figure 3 shows the best result obtained in the study with 24 experimental dogs, where the systemic concentration of lactate was increased by a balanced co-infusion of lactic acid and sodium lactate (to avoid changes in plasma pH). Lactate has been shown to be a potent inhibitor of the intestinal synthesis of citrulline (from proline or glutamine, via ornithine). While relatively successful, this approach still did not match the results obtained with crude liver extract.

### Low-temperature liver extract purification by selective solvent precipitation of hemoglobin

Revisiting the early success with simple preparation (on ice) of the crude liver extract, the inventors looked at the thermostability of other enzymes involved in arginine metabolism, and more specifically those of the urea cycle. Argininosuccinate synthase (ASS) that converts citrulline into argininosuccinate in the urea cycle at the same rate that arginase converts arginine into ornithine (and urea) was the prime suspect. When exposed to a temperature above 44°C, it will rapidly lose its activity, denature and precipitate.

The inventors then turned to possible ways of partial purification of the liver extract at low temperatures. Hemoglobin is the main protein present in the liver at high concentrations that can cause kidney failure when infused in circulation over an extended time. Several published methods of hemoglobin removal from blood plasma were tested, including precipitation with salts. The best results were obtained using an ethanol-butanol mixture, which, under appropriate conditions of temperature and time, could eliminate most of the hemoglobin, without undue loss of activity of ARG and ASS. Precipitates after the first step of tissue homogenization and after precipitation by ethanol-butanol were removed by highspeed centrifugation. Alcohols were removed by successive dilutions and concentrations on a 10 kDa ultrafilter, while the largest molecules were excluded by filtration through a 500 kDa filter. Suitable steps of preparation are shown in Figure 4.

A pilot test in a dog with lymphoma during four consecutive days was performed using 4 different porcine liver extract preparations. Three different preparations used on day 1, day 2, and day 4 were all prepared at high temperatures and did not come close to lowering plasma arginine to the desired level of < 1µM. The extract prepared cold (standard refrigerator range of 5 to 8°C, or on the ice at 0°C) led to arginine reduction to below detection, as shown by a chromatogram in Figure 5.

Having observed, understood, and explained many of the issues of arginine depletion as a treatment for cancer, the inventors have, as stated above, taken steps to perform experiments with recombinant arginase (in monomeric form) and argininosuccinate synthase (as a tetramer) in preparation for the safety and efficacy studies towards clinical approvals, initially for veterinary use and ultimately for human use. While the use of enzymes of animal origin (in this case from the porcine liver), is burdened with additional complications and regulatory restrictions, it remains a viable option considering the benefits it could bring to the treatment of cancer.

More particularly, the inventors intend to perform experiments and have ordered production of human ARG1 in *E. coli,* modified by a histidine tag (underlined) for affinity purification, of the following sequence (SEQ ID NO:1), 331 amino acids long:

MGHHHHHHGSSAKSRTIGIIGAPFSKGQPRGGVEEGPTVLRKAGLLEKLKEQ
ECDVKDYGDLPFADIPNDSPFQIVKNPRSVGKASEQLAGKVAEVKKNGRISLV
LGGDHSLAIGSISGHARVHPDLGVIWVDAHTDINTPLTTTSGNLHGQPVSFLLK
ELKGKIPDVPGFSWVTPCISAKDIVYIGLRDVDPGEHYILKTLGIKYFSMTEVDR

LGIGKVMEETLSYLLGRKKRPIHLSFDVDGLDPSFTPATGTPVVGGLTYREGLY
ITEEIYKTGLLSGLDIMEVNPSLGKTPEEVTRTVNTAVAITLACFGLAREGNHKP
IDYLNPPK.

The predicted molecular weight of this protein is 35,920 Da.

Further, the inventors intend to perform experiments and have ordered production of human ASS1 in *E. coli,* modified by a histidine tag (underlined) for affinity purification of the following sequence (SEQ ID NO:2), 424 amino acids long:

MGHHHHHHHHGSGSSKGSVVLAYSGGLDTSCILVWLKEQGYDVIAYLANIGQ
KEDFEEARKKALKLGAKKVFIEDVSREFVEEFIWPAIQSSALYEDRYLLGTSLA
RPCIARKQVEIAQREGAKYVSHGATGKGNDQVRFELSCYSLAPQIKVIAPWRM
PEFYNRFKGRNDLMEYAKQHGIPIPVTPKNPWSMDENLMHISYEAGILENPKN
QAPPGLYTKTQDPAKAPNTPDILEIEFKKGVPVKVTNVKDGTTHQTSLELFMYL
NEVAGKHGVGRIDIVENRFIGMKSRGIYETPAGTILYHAHLDIEAFTMDREVRKI
KQGLGLKFAELVYTGFWHSPECEFVRHCIAKSQERVEGKVQVSVLKGQVYIL
GRESPLSLYNEELVSMNVQGDYEPTDATGFININSLRLKEYHRLQSKVTAK.

## Claims

1. A medicament comprising a first active agent, which is an arginine-decomposing enzyme, and a second active agent, which is a citrulline-converting enzyme.

2. The medicament of claim 1, wherein the first active agent is an arginase (ARG), particularly selected from human arginase-1 (ARG1) or human arginase 2 (ARG2) and more particularly human ARG1.

3. The medicament of claim 1 or 2, wherein the arginase is present as a monomeric polypeptide.

4. The medicament of any one of the preceding claims, wherein the second active agent is an argininosuccinate synthase (ASS), particularly selected from human argininosuccinate synthase (ASS1).

5. The medicament of any one of the preceding claims, wherein the argininosuccinate synthase is present as a tetrameric polypeptide.

6. The medicament of any one of the preceding claims, wherein the first active agent and/or the second active agent are recombinant polypeptides.

7. The medicament of any one of the preceding claims, wherein the first active agent and/or the second active agent are present in an at least partially purified mammalian liver extract.

8. The medicament of any one of the preceding claims, wherein the ratio of enzymatic activities of ARG to ASS is within the range of about 0.2:1 to about 1:5, particularly of about 0.5:1 to about 1:2 and more particularly about 1:1 (expressed as enzymatic activity at concentrations of respective substrates normally present in plasma).

9. The medicament of any one of the preceding claims, wherein the daily dose of an arginase, e.g., human ARG1, in a human is about 150 to about 3000 U/kg/day, preferably about 500 to about 2000 U/kg/day, and most preferably about 1500 U/kg/day

10. The medicament of any one of the preceding claims, wherein the daily dose of an argininosuccinate synthase, e.g. human ASS1, in a human is about 0.15 to about 3 U/kg/day, preferably about 0.5 to about 2 U/kg/day, and most preferably about 1.5 U/kg/day.

11. The medicament of any one of the preceding claims, which is a single pharmaceutical preparation comprising the first and the second active agent or a combination of two separate pharmaceutical preparations.

12. The medicament of any one of the preceding claims for use in a method for the treatment of cancer including a blood cancer or a solid cancer particularly selected from hepatocellular carcinoma, melanoma, colon carcinoma, leukemia, lymphoma, osteosarcoma, soft tissue sarcoma, mast cell tumor, pancreatic cancer, lung cancer, and breast cancer.

13. The medicament of any one of the preceding claims which is administered parenterally.

14. The medicament of any one of the preceding claims which is administered by infusion.

15. The medicament of any one of the preceding claims which is administered together with insulin, particularly together with an insulin-glucose clamp.
